# EUROPEAN PATENT APPLICATION

(11) **EP 4 685 231 A1**
(43) Date of publication of application: **28.01.2026**
(21) Application number: 23928197.5
(22) Date of filing: 04.06.2023
(51) Int. Cl.: C12N 9/10, A61P 29/00, A61P 3/00, C12N 15/54, C12N 15/70, C12N 5/078, A61K 38/20, A61K 47/68, A61P 35/00

(54) **HELICOBACTER PYLORI FUCOSYLTRANSFERASE MUTANT**

(30) Priority: 23.03.2023 CN 202310292697
(71) Applicant: Ray Medicine Biotechnology Co., Ltd, Beijing 100089 (CN)
(72) Inventor: WANG, Jishu, Beijing 100094 (CN); ZHANG, Kefan, Beijing 100094 (CN); HUANG, Tao, Beijing 100094 (CN)
(74) Representative: dompatent
(86) International application number: PCT/CN2023/098183
(87) International publication number: WO 2024/192890

(57) **Abstract**

Disclosed in the present invention is a mutant of Helicobacter pylori α-1,3-fucosyltransferase, a polynucleotide encoding the mutant, and an expression vector and a host cell comprising the polynucleotide. Further disclosed in the present invention are a method for labeling a target molecule on a target cell or a target protein by using the mutant of Helicobacter pylori α-1,3-fucosyltransferase, a cell or protein labeled according to the method, and a use of the cell or protein in the preparation of a disease treatment drug. Compared with the wild type, the mutant of Helicobacter pylori α-1,3-fucosyltransferase provided by the present invention can label a target molecule on the surface of a cell membrane with better efficiency, and a method for quantitative detection of enzyme activity and an application prospect of a labeled cell or protein in the preparation of a disease treatment drug are provided.

## Description

### TECHNICAL FIELD

The present disclosure discloses a mutant of fucosyltransferase, belonging to the field of enzyme engineering technology.

### BACKGROUND TECHNOLOGY

Glycosylation is a common post-translational modification that involves the addition of sugars to amino acids or lipids under the control of enzymes. Unlike the biosynthetic pathways of proteins and nucleic acids, glycosylation is a post-translational modification of proteins , which is not driven by template. Fucosylation is a common glycosylation modification in organisms, widely present on the plasma membrane of various cell surfaces, and is considered the endpoint of glycosylation processes. Fucosyltransferase is an enzyme that transfers L-fucose from GDP-fucose (guanosine diphosphate fucose) to polysaccharides, participating in the synthesis of terminal polysaccharide structures. According to the position of fucose at the polysaccharide junction, fucosylation can be divided into core fucosylation and branched fucosylation. The α -1,3-fucosyltransferase of Helicobacter pylori catalyzes branched fucosylation, transferring fucose from GDP-fucose (donor substrate) to GlcNAc (N-acetylglucosamine) and LacNac (N-acetyllactosamine) (acceptor substrate) on the N-polysaccharide chain. Therefore, fucosyltransferase recognizes both donor and acceptor substrates and catalyzes the transfer of fucose. According to various literature reports, the main donor substrate for fucosyltransferase is the GDP-fucose with relatively small molecular weight. The enzyme activity detection kit uses high-purity GDP -Fucose as the donor substrate, and the receptor substrate is usually a purified single protein such as fetal globulin (detection kit such as Promega, Cat# VA1090, VA1091, VA1092), with enzyme activity (Km) of about 1-100 µ M. When the donor substrate becomes a GDP-Fucose derivative with large molecular weight, or the acceptor substrate is a complex protein mixture (such as the cytoplasmic membrane), the enzyme activity (Km) is very low, and the commercial detection kit mentioned above cannot detect enzyme activity.

Wu et al. utilized the fucosyltransferase of Helicobacter pylori to transfer antibody-like macromolecular proteins to polysaccharides on the cell membrane surface, such as LacNAc and α 2,3 sialylLacNAc. Using this technique, Wu et al. constructed two types of engineered cells - using natural killer cell lines (NK-92MI) and mouse primary CD8+OT-1T cells, Her2 antibodies and PD-L1 antibodies were transferred to NK-92MI and CD8+OT-1T cells, respectively, by fucosyltransferase, and specific tumor targeting and inhibitory signals against tumor cells were demonstrated in a mouse model (see Li J et al. ACS Cent Sci. 2018 Dec 26; 4 (12): 1633-1641.). Therefore, using fucosyltransferase to label molecules with therapeutic significance onto cells will significantly enhance the effectiveness of cell therapies such as CAR-T. The inventor found in this research that compared to small molecule of GDP-Fucose, fucosyltransferase reduces the enzyme activity of large molecule donor substrates by nearly a thousand times, which cannot meet the needs of engineering cell preparation.

### SUMMARY OF THE DISCLOSURE

### Technical Problem

Although several patent documents in this field have described that some substitution mutations or combinations of substitution mutations can enhance the activity of Helicobacter pylori's α-1,3-fucosyltransferase, such as CN201611051529.1 and CN202110273518.2, experiments have found that these mutants, such as mutant A128N15 (A128N, H129E, S46F, Y132I), are not obviously expressed in Escherichia coli BL21 (DE3); Alternatively, it can be expressed as inclusion bodies, such as mutants (A78S, T88A, G95V), mutants (F9Y, V19A), mutants (G224K, V284L). The enzymes expressed in inclusion bodies are not easily renaturated, and the enzyme activity is very low after renaturation (data not shown). The research and industrial value of the above mutants is relatively low. Preparation of engineering cell using fucosyltransferase as a tool enzyme requires the enzymes to better recognize complex substrates.

Therefore, the purpose of the present disclosure is to obtain highly active mutants that can recognize complex substrates and can be expressed soluble in E. coli, further to enhance the activity of Helicobacter pylori α-1,3-fucosyltransferase on macromolecular donor substrates and complex receptor substrates, and provide corresponding detection methods to expand the possibility and affordability of engineering cell preparation.

### Solution to Technical problem

Based on the above objectives, the present disclosure first provides a mutant of Helicobacter pylori α-1,3-fucosyltransferase, wherein the α-1,3-fucosyltransferase mutant mutates at amino acid positions 108 and/or 139 of SEQ ID NO.1 which refers to the wild-type Helicobacter pylori α-1,3-fucosyltransferase. To obtain the above-mentioned Helicobacter pylori α-1,3-fucosyltransferase, the technical solution adopted by the present disclosure is described as below:

Using the principle of error prone PCR mutation, based on the truncated form (sequence as shown as SEQ ID NO. 1) of Helicobacter pylori alpha-1,3-fucosyltransferase (strain ATCC 700392/26695), random substitution mutants of Helicobacter pylori's α-1,3-fucosyltransferase are obtained. SEQ ID NO. 1 is named as wild-type (wt) in the present disclosure. The wild-type and mutant were induced for expression in Escherichia coli, respectively. soluble mutants were selected for purification.

In a preferred embodiment of the α-1,3-fucosyltransferase mutant of Helicobacter pylori, the sequence of the mutant is shown in SEQ ID NO.5. Compared to the wild type, the alanine (Alanine) at position 108 in the mutant is replaced with valine (Valine) , and is named "A108V" in the present disclosure.

The present disclosure also provides a polynucleotide encoding the above-mentioned preferred embodiment of α-1,3-fucosyltransferase mutant of Helicobacter pylori, wherein the sequence of the polynucleotide is shown as SEQ ID NO.6.

The present disclosure also provides an expression vector for expressing the above-mentioned α-1,3-fucosyltransferase mutant of Helicobacter pylori, wherein the expression vector contains the above-mentioned polynucleotide. In a specific embodiment of the present disclosure, the vector is commercially available MilliporeSigma ^{™} pET-41a(+) DNA Vector, Other conventional expression vectors in the field of genetic engineering can also be applied to the present disclosure.

The present disclosure also provides a host cell for expressing the aforementioned Helicobacter pylori α-1,3-fucosyltransferase mutant, wherein the host cell contains the aforementioned expression vector. In a specific embodiment of the present disclosure, the host cell is Escherichia coli BL21 (DE3), and other conventional host cells in the field of genetic engineering can also be applied to the present disclosure.

In another preferred embodiment for the α-1,3-fucosyltransferase mutant of Helicobacter pylori, the sequence of the mutant is shown as SEQ ID NO.7. Compared to the wild type, the aspartic acid Aspartic acid at position 139 in the mutant is replaced with glutamic acid Glutamic acid. The mutant mentioned in the present disclosure is named "D139E".

The present disclosure also provides a polynucleotide encoding the above-mentioned preferred embodiment of α-1,3-fucosyltransferase mutant of Helicobacter pylori, wherein the sequence of the polynucleotide is shown as SEQ ID NO.8.

The present disclosure also provides an expression vector for expressing the above-mentioned α-1,3-fucosyltransferase mutant of Helicobacter pylori, wherein the expression vector contains the above-mentioned polynucleotide. In a specific embodiment of the present disclosure, the vector is commercially available MilliporeSigma ^{™} pET-41a(+) DNA Vector, Other conventional expression vectors in the field of genetic engineering can also be applied to the present disclosure.

The present disclosure also provides a host cell for expressing the aforementioned Helicobacter pylori α-1,3-fucosyltransferase mutant, wherein the host cell contains the aforementioned expression vector. In a specific embodiment of the present disclosure, the host cell is Escherichia coli BL21 (DE3), and other conventional host cells in the field of genetic engineering can also be applied to the present disclosure.

Secondly, the present disclosure provides a method for labeling target molecules onto target cells or protein via using the mutant of Helicobacter pylori α-1,3-fucosyltransferase as described above, the method includes:
(1) Conjugate GDP-fucose with target molecules to obtain a linkage complex;
(2) Incubate the linkage complex obtained in step (1) with the target cell or protein containing GlcNac receptor molecules in the presence of a mutant of Helicobacter pylori α-1,3-fucosyltransferase, to obtain the target cell or protein labeled with the target molecules.

In a preferred embodiment, the target molecule is a therapeutic molecule. In a specific embodiment of the present disclosure, the therapeutic molecule is a Fc fusion protein derivative of human IL-15 (referred to as N803 in the present disclosure). In the specific embodiment, GDP-fucose is coupled to purified N803 by crosslinking N-hydroxysuccinimide ester (NHS ester) with amine, Obtaining N803-GDP-fucose coupling molecules (with a molecular weight of approximately 120KD; compared to GDP-fucose derivatives with a molecular weight of 500D, referred to as high molecular weight of GDP-fucose derivatives) as donor substrates, the coupled N803 will carry about 6 to 10 GDP-fucose derivatives. In the specific embodiment, the target cells containing GlcNac receptor molecules are washed human red blood cells. Using washed human red blood cells as receptor substrates, the mutant of Helicobacter pylori α-1,3-fucosyltransferase provided by the present disclosure was used as a catalyst to label GDP-fucose - oupled N803 on the surface of red blood cell membranes.

Thirdly, the present disclosure provides a labeled cell or protein according to the above method. In a specific embodiment of the present disclosure, the red blood cells labeled with GDP - fucose-coupled N803 on the cell membrane surface were obtained. N803 is an IL-15 derivative that can activate T lymphocytes, B lymphocytes and NK cells, and mediate their proliferation and survival. It plays an important role in anti-tumor, pro-inflammatory, and anti infective activities. N803 was developed by Altor BioScience. In a phase I clinical trial, Nivolumab (PD-1 antibody, Opdivo) combined with N803 was shown to significantly prolong long-term survival in patients with metastatic non-small cell lung cancer. N803, as a recombinant protein drug, extends the serum half-life of IL-15 from a few minutes to 8-10 hours by fusing with the Fc fragment of immunoglobulin. In vivo studies on mice have shown a significant improvement in tumor treatment efficacy (Xu H, et al. A novel multimeric IL15/IL15Rα-Fc complex to enhance cancer immunotherapy. Oncoimmunology. 2021 Mar 11;10:1893500.). Compared to other tumor immunotherapy drugs, the half-life still needs to be further extended. The lifespan of mature red blood cells in humans is about 120 days. Coupling N803 to the surface of red blood cell membranes is expected to significantly increase the half-life of drugs and reduce the frequency of intravenous infusion and the dosage of drug.

Finally, the present disclosure provides the application of the aforementioned cells or proteins in the preparation of disease therapeutic drugs. By coupling drugs to the surface of mature red blood cell membranes, the half-life, distribution, metabolism, and excretion of drugs can be altered to maximize their therapeutic properties and reduce side effects. In addition, various therapeutic drugs, including peptides, small molecules, and nucleic acids, can be conjugated onto the cell membranes of NK cells, T cells, B cells, as well as stem cells and precursor cells through the method provided by the present disclosure. This not only fully preserves the functions of each cell, but also leverages the characteristics of conjugated drugs to achieve targeted therapy.

In a preferred embodiment, the disease is a tumor, inflammatory disease, metabolic disease, or rare disease requiring enzyme replacement therapy. For example, coupling antibodies against tumor associated antigens (TAAs) such as anti-Her2, EFGR, VEGFR, CD19, etc. to the surface of NK cells enhances their targeting and improves their anti-tumor efficacy. In addition, coupling one or several tumor specific recognition antigens can improve the targeting of CAR-T and reduce off target side effects of CAR-T. Furthermore, metabolism-related enzymes can be coupled to the surface of blood cells, such as uric acid oxidase, to clear uric acid from the blood and tissues and treat refractory gout. In addition, coupled with immune cell suppressive antibodies or peptides such as PD-L1, the pathologically activated T and B lymphocytes is inhibited, thereby autoimmune diseases such as lupus erythematosus is treated; Or activate Treg cells to treat inflammatory diseases such as rheumatoid arthritis.

### Beneficial Effects of the present disclosure

The present disclosure utilizes the principle of error prone PCR mutation to screen and obtain randomly substituted mutants A108V and D139E of Helicobacter pylori alpha-1,3 fucosyltransferase based on the alfa-1,3 fucosyltransferase truncated form of wild-type Helicobacter pylori. Under the same substrate and experimental conditions, mutants D139E and A108V exhibited higher catalytic efficiency in coupling N803 onto the cell membrane surface, with catalytic efficiency 1.75-1.82 times that of the wild type. However, the other mutants (N167Q and P64S) showed a significant decrease, only 25% -6% of the wild type. The Western blot method also confirmed that mutant D139E and mutant A108V catalyze more N803 on red blood cells. Through semi-quantitative comparison of grayscale analysis, mutant D139E and mutant A108V carry 2 times and 5 times more N803 than the wild type, respectively. The mean fluorescence intensity (MFI) of flow cytometry was used as an indicator of enzyme catalytic reaction products to quantitatively evaluate the enzyme activity of the mutant. The Km of mutant D139E was about 1 µ M, and the enzyme activity was three times higher than that of the wild-type Km (about 3 µ M).

### BRIEF DESCRIPTION OF THE DRAWINGS

Figure 1. List of mutations generated by error prone PCR, amino acids 1-120 (excluding mutations of stop codon and frameshift);
Figure 2. List of mutations generated by error prone PCR, amino acids 121-240 (excluding mutations of stop codon and frameshift);
Figure 3. List of mutations generated by error prone PCR, amino acids 241-364 (excluding mutations of stop codon and frameshift);
Figure 4. Detection diagram of soluble expression of mutants in Escherichia coli;
Figure 5. Flow cytometry detection diagram of human red blood cells labeled with macromolecular donor substrate catalyzed by wild-type and mutant ;
Figure 6. Comparison diagram of mean fluorescence intensity of human red blood cells labeled with macromolecular donor substrates catalyzed by wild-type and mutant;
Figure 7. Western blot analysis diagram of the relative loading of human red blood cells labeled with macromolecular donor substrates catalyzed by wild-type and mutant;
Figure 8. Comparison diagram of enzyme activity between wild-type and mutant in a simple system (high-purity GDP-Fuchose as donor substrate, fetal globulin as acceptor substrate);
Figure 9. Comparison diagram of mutant D139E in different concentrations transferring donor (GDP-Fucose derivative) to acceptor (cell membrane surface of red blood cells) detected by flow cytometry under the condition of complex donor and acceptor substrates;
Figure 10. The relationship diagram between mean fluorescence intensity (MFI) and mutant D139E concentration in flow cytometry;
Figure 11. Comparison diagram of enzyme activity between wild-type, mutant D139E, and mutant N167Q by flow cytometry under conditions of complex donor and acceptor substrates.

### SPECIFIC EMBODIMENTS

The present disclosure is further described with specific examples below, and the advantages and features of the present disclosure will become clearer with the description. However, these examples are only exemplary, and do not constitute any further limitations to the scope defined by the claims of the present disclosure.

### Example 1. Preparation of α -1,3-fucosyltransferase mutants of Helicobacter pylori

### 1. Screening for mutants

Error prone PCR is a method that mutants is randomly introduced into target genes at a certain frequency by adjusting reaction conditions when amplifying target genes using DNA polymerase to obtain random mutants of protein molecules.

In the present disclosure, the protein sequence of the wild-type α-1,3-fucosyltransferase of Helicobacter pylori is shown as SEQ ID NO. 1. Suzhou Junji Biotechnology Co., Ltd. is commissioned to synthesize template DNA according to the sequence SEQ ID NO. 2 and corresponding primers:
Forward: 5'-CATATGTTTCAGCCGCTGCTG-3'
Reverse: 5'-GTTTACGCGTAAGTCATCGTAATTG-3'
The reaction conditions for error prone PCR are as follows:

Add 5 µ L of 10 × TITANUM Taq Buffer (400 mM Tricine KOH (pH 8.0 at 25 ° C), 160 mM KCl, 35 mM MgCl2, 37.5 µ g/mL BSA), 1 µ L of dGTP (2 mM), 1 µ L of 50 × Diversified dNTP Mix (10 mM dATP, dCTP, dGTP, and dTTP respectively), 10 µ M of each primer, 50 ng of DNA template, and 20 units of TITANUM Taq to a 50 µ L PCR system. Fill the PCR grade pure water to 50 µ L of final volume. The above reagents are from Clontech, Cat# 639141.

After thoroughly mixing and centrifuging the reaction components, place the PCR tube into the PCR thermal cycle, and set the PCR reaction parameters as 94 ° C for 30 seconds; 25 cycles (94 ° C for 30 seconds, 68 ° C for 1 minute); 68 ° C for 1 minute; Soak at 4 ° C.

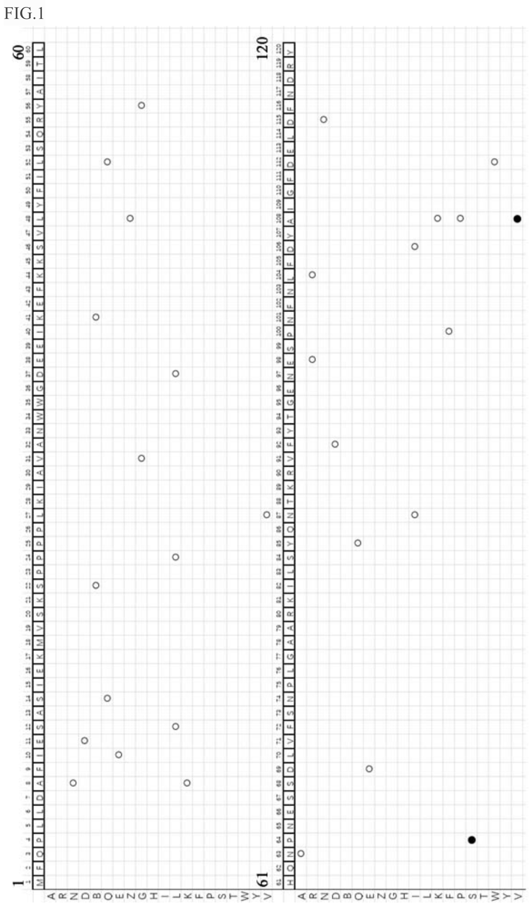

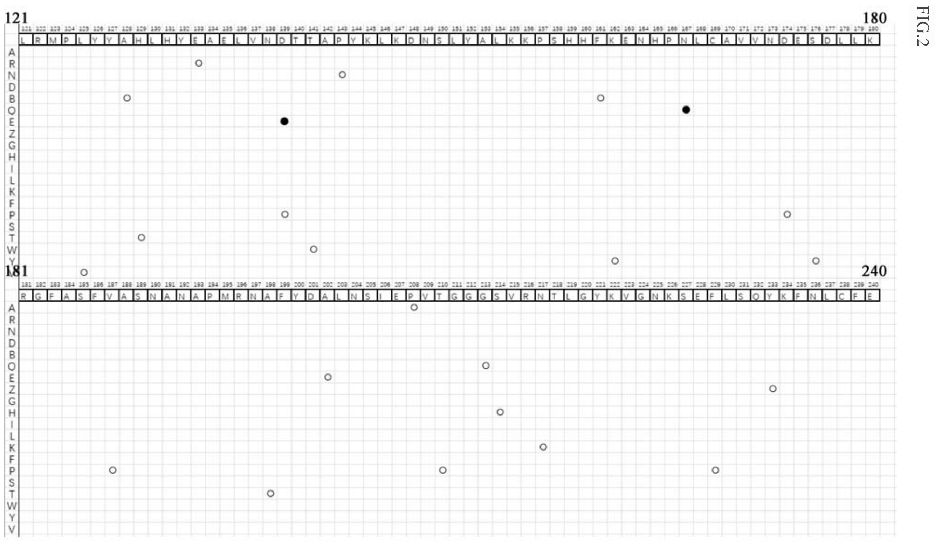

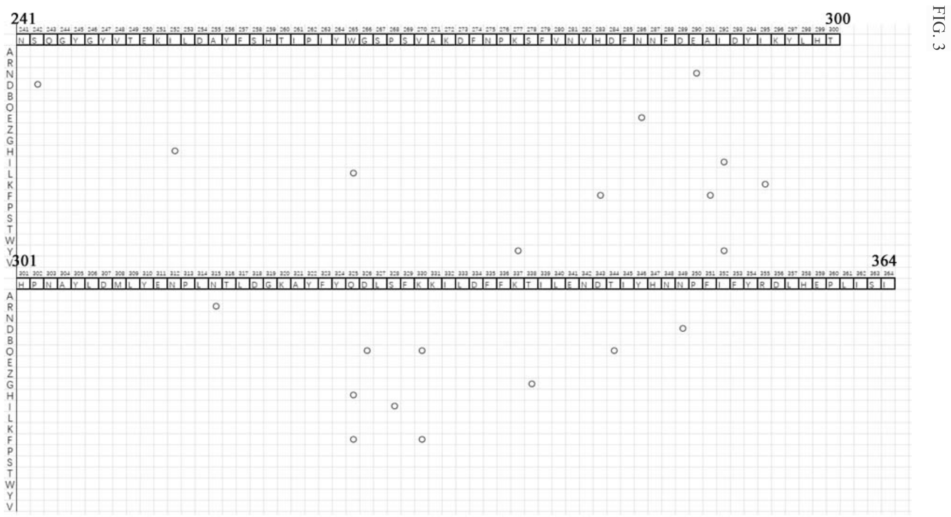

take the products obtained from the above PCR on agarose electrophoresis, cut the gel to recover and purify, and then according to a 10 microliter volume reaction system: take 1 microliter (50ng) of T vector (T vector is pMD ^{™} 19-T Vector Cloning Kit, Takara Cat. No. 6013), with equimolar PCR products added. Add 1 µ L of 10 × Buffer containing ATP, appropriate units of T4 DNA ligase, and make up to 10 µ L with ddH₂O. Link overnight at 16 °C. Transform the linked product into Escherichia coli, as described below:
(1) Preheat LB plates containing Amp, X-Gal, and IPTG at 37 °C.
(2) Add 10 microliters of linker product to 100 microliters of competent cells and ice bath for 30 minutes.
(3) Transfer the centrifuge tube to 42 °C water bath, heat shock for 90 seconds, then do not shake the centrifuge tube and quickly place it on ice for 2 minutes.
(4) Add 300 microliters of SOC culture medium to the centrifuge tube, mix well with the pipette tip, and gently shake at 37 °C and 150rpm for 60 minutes.
(5) Evenly coat 200 microliters of transformed bacterial solution onto LB plates containing 50mg/ml Amp, 20mg/ml X-gal, and 200mg/ml IPTG, invert at 37 °C overnight, and select white colonies for sequencing commissioned by Suzhou Junji Biotechnology Co., Ltd. Based on the sequencing results, remove nonsense mutations, frameshift mutations, and stop codon mutations, and select single mutation site. Then, DNA synthesis was carried out one by one and cloned into pET41b (MilliporeSigma) ^{™} pET-41a(+) DNA Vector, Catalog number: 70-556-3). The list of mutants is shown in Figure 1-3. In Figure 1-3, the amino acid sequence of Helicobacter pylori o-1,3-fucosyltransferase are arranged horizontally one by one, and the 20 types of essential amino acids for the human body is in vertical direction. The horizontal axis in the intersection point corresponds to the wild-type amino acid site, and the vertical axis represents the mutation caused by error prone PCR.

### 2. Expression of mutants

The expression of the α-1,3-fucosyltransferase mutant of Helicobacter pylori is briefly described as follows:
(1) Take 2 microliters of plasmid and add it to 100 microliters of BL21 (DE3) competent cells (ThermoFisher, catalog number: EC0114), mix immediately, and place on ice for 30 minutes.
(2) Heat shock at 42 °C for 90 seconds and quickly ice bath for 2 minutes.
(3) Add 500 uL of LB medium, incubate at 37 °C, rpm<=200, and shake for 60 minutes.
(4) Centrifuge at 6000rpm for 1 minute, discard most of the supernatant, keep about 100-150 uL, resuspend the bacterial cells, coat them on LB plates containing Amp, and incubate overnight at 37 °C.
(5) Expression in small amount: Select one monoclonal and culture it in 1 mL of LB medium with Amp resistant at 37 °C and 220 rpm for about 5 hours. Add 2.5 mL of LB liquid medium containing Amp to the tube in previous step and culture it overnight at 37 °C and 220 rpm.
(6) Transfer the overnight-cultured bacterial solution to 20ml LB medium containing Amp at a ratio of 1:50, at 37 °C and 220rpm, and culture until OD600=0.6 (about 3h). Add a final concentration of 0.5mM IPTG and culture at 30 °C and 220rpm for 6h.
(7) Measure the OD600 of the culture medium, take 10OD bacterial solution, centrifuge at 10000rpm for 2 minutes, and remove the supernatant.
(8) Resuspend the bacterial cells in 1 mL of lysis buffer (10 mM Tris HCl, pH 8.0) and place them on ice for ultrasonic lysis of cells. Ultrasonic conditions: 130W, 4 min, on 3s, off 3s.
(9) After ultrasound treatment, the lysate was centrifuged at 12000rpm for 10 minutes to obtain the supernatant;( lysis supernatant), Centrifuge the supernatant at a speed of 125000g at 4 ° C.

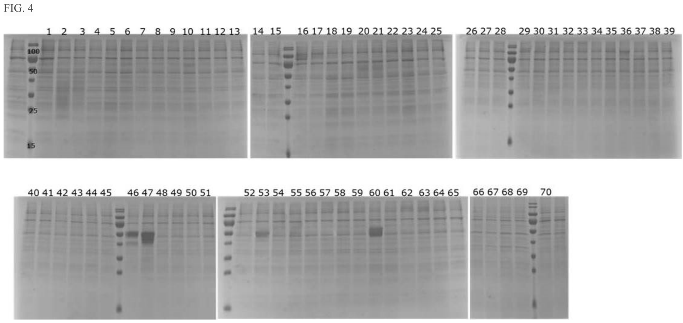

Figure 4 shows the SDS-PAGE analysis on the soluble portion of inducted expression of all mutants in Escherichia coli. 20 microliters of 5 × Reduce loading buffer were added to 80 microliters of purified recombinant protein, then heated at 95 °C for 5 minutes, and 12.5 microliters (equivalent to 0.1OD) of each sample were taken for SDS-PAGE electrophoresis. 50 microliters of 1 × Reduce loading buffer was added to the insoluble portion and heated at 95 °C for 5 minutes, afterwards, SDS-PAGE electrophoresis was also performed (results not shown). The SDS-PAGE electrophoresis results showed that only four mutants, P64S, A108V, D139E, and N167Q, had visible soluble expression. Samples 46, 47, 53, and 60 in Figure 4 are the four mutants P64S, A108V, D139E, and N167Q, respectively. Among them, P64S refers to the substitution of proline (Proline) at position 64 by serine (Serine) ; A108V refers to the substitution of alanine (Alanine) at position 108 by valine (Valine); D139E refers to the substitution of aspartic acid (Aspartic acid) at position 139 by glutamic acid (Glutamic acid); N167Q refers to the substitution of Asparagine (Asparagine) at position 167 by Glutamine (Glutamine).

### 3. Purification of Enzyme Proteins

According to the above conditions for expression induction, expand the culture volume to 100ml and obtain the supernatant of the ultrasonic lysis solution. According to the user manual, apply the supernatant to the HiTrap chelating HP column and elute with 20mM imidazole solution. The eluant was collected and the solvent was replaced with 50 mM Tris buffer solution (pH 8.0) through dialysis, then further purified by gel filtration chromatography (superdex200, GE Healthcare) to obtain a protein with high purity, producing a protein with more than 98% homogeneity. Using bovine serum albumin as a standard, the protein concentration was determined using the Bio-Rad protein assay kit based on the Bradford method.

### Example 2. Preparation of macromolecular donor substrates

N803 (also known as ALT803, is a Fc fusion protein derivative of human IL-15) [2013 The IL 15 based superagonist ALT 803 promotes the antigen independent conversion of memory CD8 T cells into innate like effector cells with antitumor], Based on the sequences published in the literature, design corresponding DNA and fuse it into the human IgG4 Fc sequence. Entrust Suzhou Junji Biotechnology Co., Ltd. to synthesize plasmid DNA and clone it into vector pRM293(pRM293 was obtained by modifying the plasmid pTT5, as detailed in Shi C. Purification and characterization of a recombinant G-protein-coupled receptor, Saccharomyces cerevisiae Ste2p, transiently expressed in HEK293 EBNA1 cells. Biochemistry. 2005;44(48):15705-15714.). And entrusted Suzhou Junji Biotechnology Co., Ltd. to transiently transfect the plasmid into HEK293 cells (National Research Council, Canada) and perform affinity purification using Mabselect sure (Protein A, GE healthcare). GDP-fucose was coupled to purified N803 via amine crosslinking with N-hydroxysuccinimide ester (NHS ester), and thereafter, unless otherwise specified, N803 was always GDP-fucose-coupled N803.
1. NHS ester-activated crosslinking agents and labeled compounds react with primary amines on N803 under physiological to weakly alkaline conditions (pH 7.2 to 9) to form stable amide bonds. Under this principle, the NHS on TCO-PEG4-NHS reacts with the primary amine on N803 protein to obtain TCO-PEG4-N803. The specific methods and processes are as follows:
   400 µg of purified N803 protein was reacted with 150 µg of TCO-PEG4-NHS (Shanghai Perry Pharmaceutical Technology Co., Ltd., product number A34125), and 20 mM HEPES buffer (pH 7.0-7.5, Thermo Fisher Scientific (China) Co., Ltd., product number 15630) was added. After incubation at room temperature for 30 minutes, 5 µ mol Tris buffer was added to terminate the reaction. Incubate at room temperature for 5 minutes, the reaction product was added to a PD SpinTrap G-25 desalination column (Cytiva, product number 28918004) and centrifuged at 800 × g to remove unreacted and reacted small molecules, obtaining pure TCO-PEG4-NHS.
2. Based on the reverse demand Diels Alder cycloaddition reaction between trans cyclooctene and tetrazine, the connection between TCO (trans cyclooctene) and Tz (Tetrazine) forms a dihydropyridazine bond. Under this principle, TCO-PEG4-N803 reacts with the derivative of GDP-fucose, GDP-Fucose-Triazole-PEG4-Tz, to obtain GDP-Fucose-Triazole-PEG4-PEG4-N803, which is the GDP - fucose-coupled N803. Specifically, as follows:

Further react the TCO-PEG4-N803 obtained in the previous step with 30 µg of GDP-Fucose-Triazole-PEG4-Tz (synthesized by Yantang Biotechnology Co., Ltd., product number YT-HJP-3-29), incubate at room temperature for 30 minutes, and add the reaction product to a PD SpinTrap G-25 desalination column (Cytiva company, product number 28918004). Centrifuge at 800 × g to remove unreacted and reacted small molecules, and obtain pure GDP-fucose-coupled N803.

According to MALDI-TOF detection, the coupled N803 carries about 6 to 10 GDP-fucose derivatives. Unless otherwise specified, N803 is a GDP-fucose-conjugated N803.

### Example 3. Flow cytometry detection on macromolecular donor substrate labeling human red blood cells catalyzed by mutant

2-3 mL of blood is obtained from anonymous healthy blood donors, with the blood collection site being the anterior cubital vein. After disinfecting the skin, a disposable vacuum blood collection device is used and operated by professionals. The blood slowly flows along the wall of the test tube, reaches the marked area, and is pulled out of the tube. It is quickly reversed several times and mixed to prevent coagulation. Centrifuge the blood at 500 × g for 5 minutes at 4 ° C and mark the hematocrit (red, lower layer) and plasma (yellow, upper layer) levels on a test tube. Slowly and thoroughly extract blood plasma and intermediate sediment (buffy coat) by a micropipette, add bleach to the extracted liquid, and discard it into biohazardous waste. Transfer 0.5 mL of red blood cell hematocrit to a 15 mL centrifuge tube, add 5 mL of PBS pH 7.4 solution and cover with a lid. Flip over several times and mix well. Centrifuge at 4 degrees Celsius for 5 minutes at 500 × g, take up the supernatant, and discard. Repeat the PBS washing steps 3-4 times. Wash the red blood cells and add 1000 microliters of PBS. Store at 4 degrees Celsius for later use.

Add 100 µg/mL of fucosyltransferase mutant and 150 µg/mL of GDP -fucosyltransferase-conjugated N803 to 5 × 10⁹/mL red blood cells, and incubate at room temperature for 30 minutes. Wash with PBS pH 7.4, centrifuge at 500 × g, and remove the supernatant. Red blood cells were resuspended in PBS pH 7.4 and incubated with anti-hIgG-Biotin antibody (Beijing Baiaolaibo Technology Co., Ltd. product number F030822, diluted 1:200) at 4 ° C for 30 minutes. Afterwards, take them washed once with PBS, stained with Streptavidin PE (BioLegend, Inc., product number 405204, diluted 1:200) and incubated at 4 ° C for 30 minutes. Afterwards, take them washed once with PBS, detected using a flow cytometer (Product model MateCyte from Beijing Cenglang Biotechnology Co., Ltd.) and analyzed using NovoExpress software.

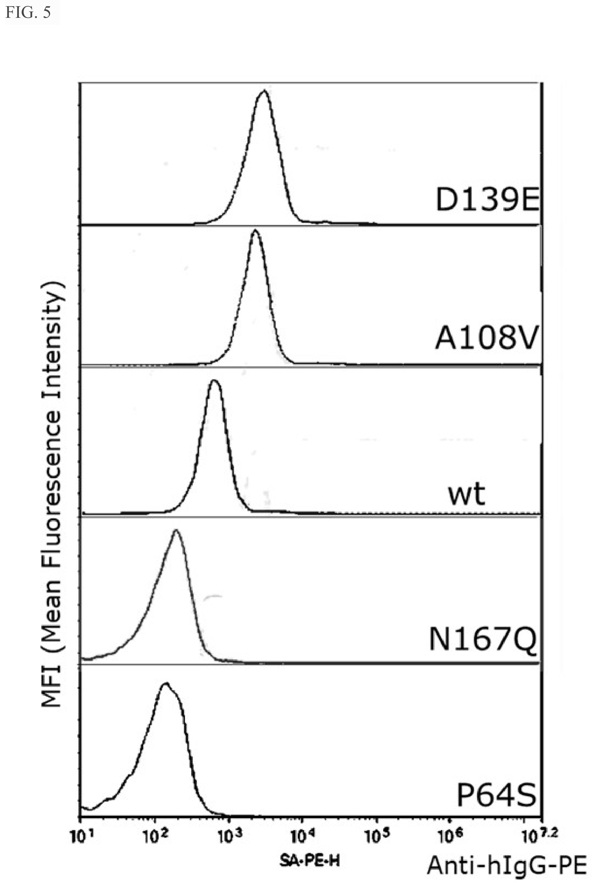

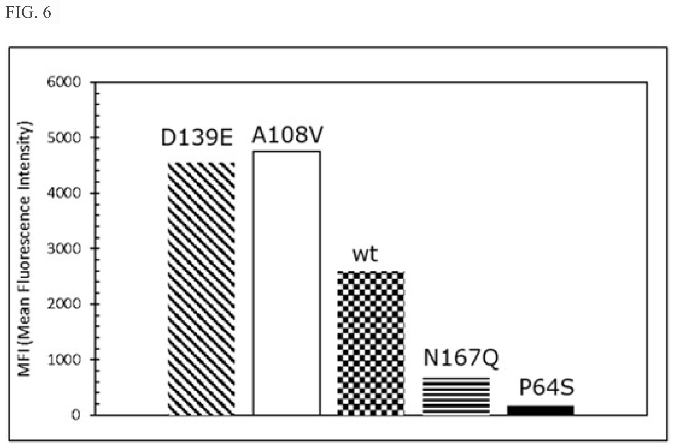

As shown in Figure 5, under the conditions of same substrate and experimental, mutant D139E, mutant A108V, mutant N167Q, mutant P64S, and wild-type were all able to conjugate GDP-Fucose-coupled N803 on the surface of red blood cell membranes, but with different efficiencies. Mutants D139E and A108V can couple more N803 onto the cell membrane surface, with an efficiency 1.75-1.82 times that of the wild type. However, mutants N167Q and P64S show a significant decrease, with only 25% -6% of the wild type (Figure 6).

### Example 4. Western blot method for detecting the presence and relative loading of antibodies on red blood cell membranes

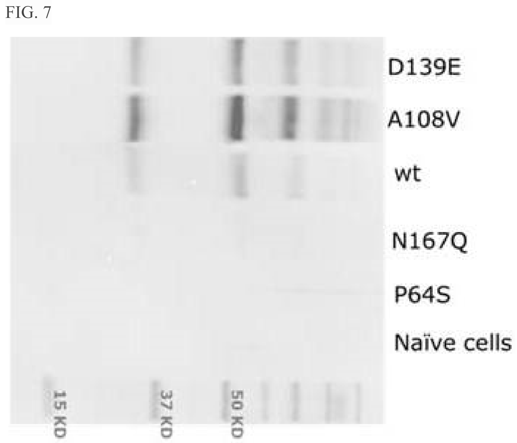

To further confirm the transferring of N803 onto the cell membrane of red blood cells, Western blot was used to detect the presence of antibodies on the red blood cell membrane and their relative loading. Red blood cells do not have nuclei or organelles and can be lysed in hypotonic solutions. After removing soluble intracellular hemoglobin, the remaining cells are membrane proteins. Under the above conditions, 5 × 10⁷ red blood cells catalyzed by mutants of different fucosyltransferases were lysed with purified water at low osmotic pressure. Membrane proteins were extracted using a cell membrane protein extraction kit (Beijing Xinquan Yongshuo Technology Co., Ltd., product ID. XQ-P1203), and cell membrane protein extraction was performed according to the kit instructions. The brief description is as follows: 500 µLCER reagent was added to every 1 × 10⁷ cell or every 100 µ L cell precipitate, shaken and resuspended, and ice bath for 2 minutes. Transfer the cell suspension to an ice-precooled glass homogenizer and homogenize manually up and down for 20-30 times in an ice water bath. Centrifuge the lysate at 800 g and at 4 °C for 5 minutes to obtain the supernatant, which is the mixture of membrane - cytoplasm. Add 1/10 volume of MER to the supernatant, mix well, ice bath for 5 minutes, 14000 rpm, 4 °C, centrifuge for 30 minutes, and the precipitate obtained is the membrane component. Resuspend with 50 µ L of resuspended buffer for future use. Add the same volume of 2 × SDS-PAGE electrophoresis buffer, take 20 µ L of protein sample separated by PAGE, and transfer it to nitrocellulose film. Incubate it with the primary antibody labeled with anti-hIgG-Biotin (Baiolebo, product number F030822;) and Streptavidin-HRP (Kingsray, product number M00091), rinse with TBST, and perform chemiluminescence reaction using the ChemiDoc ^{™} imaging system (BioRAD) was used for imaging, and untreated red blood cells were labeled as Na ï ve cells as negative controls (Figure 7). Figure 7 shows that the presence of N803 was not detected on red blood cells without enzyme treatment, and no significant N803 was detected on red blood cells catalyzed by mutant N167Q and mutant P64S. N803 can be detected on red blood cells catalyzed by mutants D139E and A108V, as well as on red blood cells catalyzed by wild-type enzymes, with more N803 carried on red blood cells catalyzed by mutants D139E and A108V. According to the image analysis software ChemiDoc Imagers provided by the imaging system for grayscale analysis, the N803 carried on mutant D139E and mutant A108V is twice and five times that of the wild type, respectively.

### Example 5. Enzyme activity detection using small molecules and simple proteins as substrates

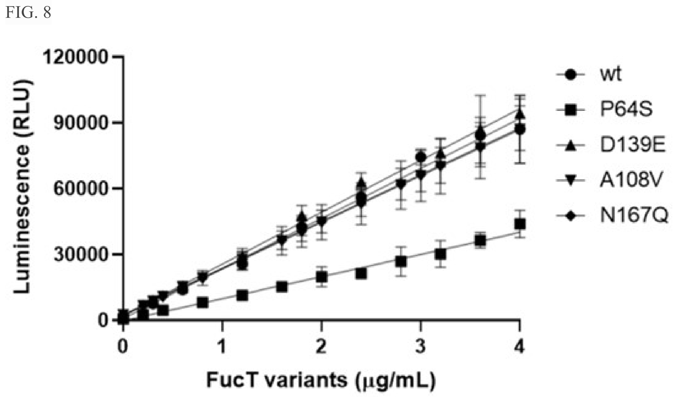

During the research process, the Promega assay kit was also used to detect the enzyme activity of four mutants using high-purity GDP-Fucose as the donor substrate and fetal globulin as the acceptor substrate. The enzyme activity was measured according to the manual (Promega GDP Glo) ^{™} Glycosyltransferase Assay ) .The 100 µM ultra pure GDP-fucose (Promega Cat. # VA1097) was used as the donor substrate, and 40 µ M fetal globulin (Promega Cat # V4961) was used as the acceptor substrate. Purified mutants of fucosyltransferase with gradient dilution were sequentially added (0ng, 5ng, 10ng, 20ng, 30ng, 40ng, 50ng, 60ng, 70ng, 80ng, 90ng, 100 ng). All enzyme reactions were performed in a 25 µ L volume of white 96 well plate. Incubate at room temperature for 60 minutes and put in GloMax ^{®} 96 microplate luminescence detector (Cat # E6501), read at room temperature, and plot according to the mass of enzymes in the reaction system as the horizontal axis and the data read on the luminescence detector as the vertical axis. Figure 8 shows that except for mutant P64S, mutant D139E, mutant A108V3, and mutant N167Q do not show significant differences in enzyme activity compared to the wild type.

Therefore, enzyme activity detection using small molecules (GDP-Fucose) and simple proteins (fetal globulin) as substrates cannot truly reflect the catalytic ability of enzymes under complex conditions.

### Example 6. Quantitative analysis of enzyme activity in complex systems using the mean fluorescence intensity (MFI) by flow cytometry as an indicator of enzyme reaction products

The mean fluorescence intensity (MFI) by flow cytometry can be used to compare the amount of target molecules on the cell membrane under the same conditions, and can serve as an indicator of enzyme reaction products. In the present disclosure, fucosyltransferase is used as a tool enzyme to transfer clinically therapeutic significant proteins onto the cell membrane. When preparing engineered cells, the substrate of fucosyltransferase is no longer a single and simple substrate under ideal conditions, but a complex and poorly homogeneous substrate mixture. For example, the donor substrate used in the present disclosure is GDP-fucosyltransferase-coupled N803, and the acceptor substrate is red blood cells. The detection established in Example 3 is to compare the amount of markers lablled on cells between wild-type and mutant under the same "single" concentration and under the same conditions (donor concentration, number of receptors). It cannot fully reflect enzyme activity, it is just a simple comparison, belonging to qualitative analysis and cannot calculate Km. It's purpose is to distinguish and remove inactive mutants. The results of Example 3 (Figure 5) and Example 5 (Figure 8) show that the enzyme mutants exhibit completely different characteristics in simple and complex systems. For example, mutant N167Q has good enzyme activity in simple systems, but no enzyme activity in complex systems. Therefore, when describing the activity characteristics of the tool enzymes required for engineering cell preparation, it is necessary to establish a quantitative analysis method for enzyme activity based on complex systems. Therefore, we established a quantitative detection method for enzyme activity based on flow cytometry, and conducted nonlinear regression analysis according to the Michaelis-Menten equation to calculate Km through the fitted equation. In this method, the first step is to find the appropriate enzyme concentration, which in the present disclosure means "not selecting saturation concentration and insensitive area", and measuring data for different concentrations of "substrates"; Then, at the enzyme concentration, data on enzyme reaction products (MFI was used as the reaction product data in the present disclosure) were measured for different concentrations of substrates (in the present disclosure, the donor substrate GDP-fucose-coupled N803 was selected). Nonlinear regression analysis was performed based on the Michaelis-Menten equation, and Km was calculated using the fitted equation. Km is only related to the properties of the enzyme and not related to the concentration of the enzyme.

Firstly, the appropriate enzyme concentration is determined. In the case of excess substrate, the enzyme concentration is gradually increased. Then, measure the efficiency that substrate is transferred to the cell and select the enzyme concentration with a moderate efficiency. The specific process is as follows:
Add 50 µ g/mL of GDP-fucose-conjugated N803 to 5 × 10⁹/mL of red blood cells, and add D139E fucosyltransferase mutant at different concentrations of 0 µ g/mL, 5 µ g/mL, 10 µ g/mL, 20 µ g/mL, 50 µ g/mL, and 100 µ g/mL, respectively. Incubate at room temperature for 30 minutes. Wash with PBS pH 7.4, centrifuge at 500 × g, and remove the supernatant. Red blood cells were resuspended in PBS pH 7.4 and incubated with anti-hIgG-Biotin antibody (product number F030822, diluted 1:200 from Beijing Baiaolaibo Technology Co., Ltd.) at 4 ° C for 30 minutes. After washing once with PBS, they were stained with Streptavidin PE (BioLegend, Inc., product number 405204, diluted 1:200) and incubated at 4 ° C for 30 minutes. After washed once with PBS, they were detected using a flow cytometer (Product model MateCyte from Beijing Cenglang Biotechnology Co., Ltd.) and analyzed using NovoExpress software. Obtain the mean fluorescence intensity (MFI) by flow cytometry.

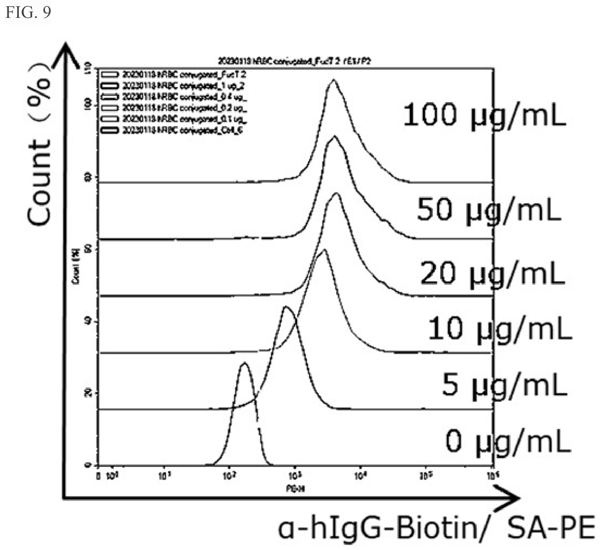

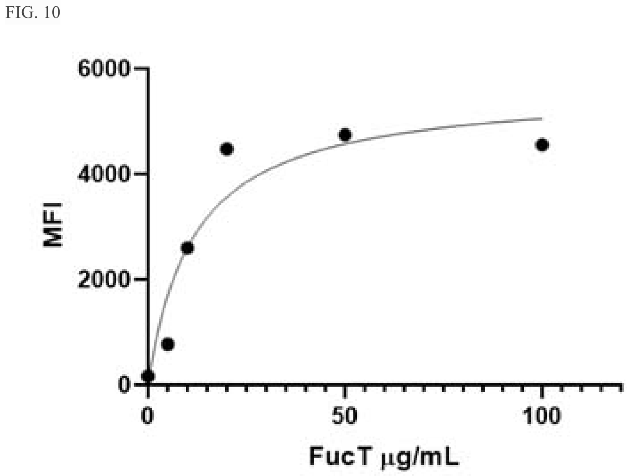

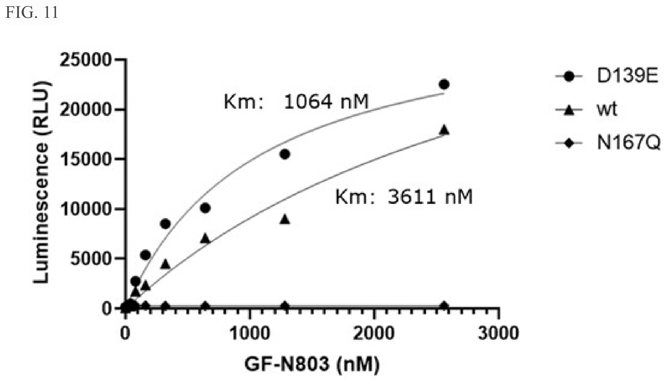

As shown in Figures 9 and 10, the reaction reached saturation at a concentration of 20 µ g/mL of the mutant D139E, and 10 µ g/mL (80 nM) was selected for Km measurement. The process is as follows:
The concentrations of wild-type, mutant N167Q, and mutant D139E were fixed at 10 µ g/mL (80 nM), and 0 nM (0 µ g/mL),~40 nM (5 µ g/mL),~80 nM (10 µ g/mL),~160 nM (20 µ g/mL), 320 nM (40 µ g/mL), 640 nM (80 µ g/mL), and 1280 nM (160 µ g/mL) of GDP-fucose-conjugated N803 were added to 5 × 10⁹/mL red blood cells. Incubate and perform flow cytometry detection using the method described in Example 3, obtain the mean fluorescence intensity by flow cytometry, and then perform nonlinear fitting using GraphPadPrism 8 software. Choose the enzyme kinetics analysis and Michaelis Menten equation provided in the software to calculate the Km. Figure 11 shows a comparison diagram of enzyme activity between wild-type, mutant D139E, and mutant N167Q measured by flow cytometry under complex donor and acceptor substrates. Mutant N167Q has almost no enzyme activity, mutant D139E has a Km of about 1 µ M, and the wild-type has a Km of about 3 µ M, the enzyme activity increased threefold compared to the wild-type Km (approximately 3 µ M).

### INDUSTRIAL APPLICABILITY

The present disclosure discloses a mutant of Helicobacter pylori α-1,3-fucosyltransferase. The mutant can be prepared industrially, showing the industrial applicability.

## Claims

1. A mutant of α-1,3-fucosyltransferase of Helicobacter pylori, wherein the mutant of α-1,3-fucosyltransferase of Helicobacter pylori mutates at amino acid positions 108 and/or 139 of SEQ ID NO.1 which refers to the wild-type Helicobacter pylori α-1,3-fucosyltransferase of Helicobacter pylori.

2. The mutant of α-1,3-fucosyltransferase of Helicobacter pylori of claim 1, wherein the sequence of the mutant of α-1,3-fucosyltransferase of Helicobacter pylori is shown as SEQ ID NO.5.

3. A polynucleotide encoding the mutant of α-1,3-fucosyltransferase of Helicobacter pylori of claim 2, wherein the sequence of the polynucleotide is shown as SEQ ID NO.6.

4. An expression vector for expressing the mutant of α-1,3-fucosyltransferase of Helicobacter pylori of claim 2, wherein the expression vector contains the polynucleotide of claim 3.

5. A host cell for expressing the mutant of α-1,3-fucosyltransferase of Helicobacter pylori of claim 2, wherein the host cell contains the expression vector of claim 4.

6. The mutant of α-1,3-fucosyltransferase of Helicobacter pylori of claim 1, wherein the sequence of the mutant of α-1,3-fucosyltransferase of Helicobacter pylori is shown as SEQ ID NO.7.

7. A polynucleotide encoding the mutant of α-1,3-fucosyltransferase of Helicobacter pylori of claim 6, wherein the sequence of the polynucleotide is shown as SEQ ID NO.8.

8. An expression vector for expressing the mutant of α-1,3-fucosyltransferase of Helicobacter pylori of claim 6, wherein the expression vector contains the polynucleotide of claim 7.

9. A host cell for expressing the mutant of α-1,3-fucosyltransferase of Helicobacter pylori of claim 6, wherein the host cell contains the expression vector of claim 8.

10. A method for labeling target molecules onto target cells or protein via using the mutant of α-1,3-fucosyltransferase of Helicobacter pylori of claim 2 or 6, wherein the method comprises:
(1) conjugating GDP-fucose with target molecules to obtain a linkage complex;
(2) incubating the linkage complex obtained in step (1) with the target cells or protein containing GlcNac receptor molecule in the presence of said mutant of α-1,3-fucosyltransferase of Helicobacter pylori, to obtain the target cells or target protein labeled with the target molecules.

11. The method of claim 10, wherein the target molecules are therapeutic molecules.

12. A labeled cell or protein according to the method of claim 11.

13. A use of the cell or protein of claim 12 in the preparation of disease therapeutic drugs.

14. The use of claim 13, wherein the disease is a tumor, inflammatory disease, metabolic disease, or rare disease requiring enzyme replacement therapy.
